# EUROPÄISCHE PATENTSCHRIFT

(11) **EP 1 383 770 B1**
(45) Veröffentlichungstag und Bekanntmachung des Hinweises auf die Patenterteilung: **22.09.2004**
(21) Anmeldenummer: 02757725.3
(22) Anmeldetag: 26.03.2002
(51) Int. Cl.: C07D 487/04

(54) **VERFAHREN ZUR HERSTELLUNG VON 4,6-DIAMINOPYRIMIDO 5,4-D]PYRIMIDINEN**
METHOD FOR THE PRODUCTION OF 4,6-DIAMINOPYRIMIDO 5,4-D]PYRIMIDINES
PROCEDE DE PRODUCTION DE 4,6-DIAMINOPYRIMIDO 5,4-D]PYRIMIDINES

(30) Priorität: 30.03.2001 DE 10115921
(43) Veröffentlichungstag der Anmeldung: 28.01.2004
(73) Patentinhaber: Boehringer Ingelheim Pharma GmbH & Co.KG, 55216 Ingelheim am Rhein (DE)
(72) Erfinder: RALL, Werner, 88441 Mittelbiberach (DE); LOCK, Ralf, 55122 Mainz (DE); BELZER, Werner, 56329 St. Goar (DE)
(86) Internationale Anmeldenummer: PCT/EP2002/003353
(87) Internationale Veröffentlichungsnummer: WO 2002/079202

(56) Entgegenhaltungen:
- WO-A-97/32880
- REWCASTLE G W ET AL: "TYROSINE KINASE INHIBITORS 12. SYNTHESIS AND STRUCTURE-ACTIVITY RELATIONSHIPS FOR 6-SUBSTITUTED 4-(PHENYLAMINO)PYRIMIDO5,4-DPYRIMIDI NES DESIGNED ASINHIBITORS OF THE EPIDERMAL GROWTH FACTOR RECEPTOR" JOURNAL OF MEDICINAL CHEMISTRY, AMERICAN CHEMICAL SOCIETY. WASHINGTON, US, Bd. 40, Nr. 12, 1997, Seiten 1820-1826, XP001056512 ISSN: 0022-2623

## Beschreibung

Die Erfindung betrifft ein Verfahren zur Herstellung von 4,6-Diaminopyrimido[5,4-*d*]pyrimidinen der Formel I, 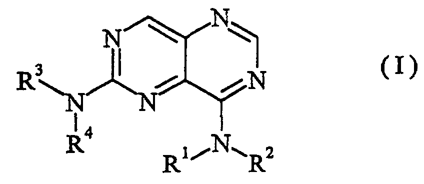 worin R¹, R², R³, und R⁴ die angegebene Bedeutung besitzen, aus einer Verbindung der Formel II, 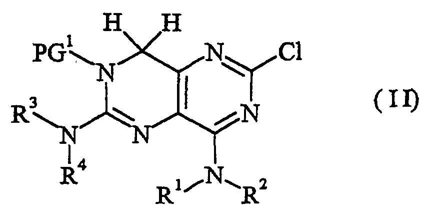 worin R¹, R², R³, und R⁴ die angegebene Bedeutung besitzen und
PG¹ für eine geeignete Schutzgruppe steht.

### Hintergrund der Erfindung

4,6-Diaminopyrimido[5,4-*d*]pyrimidine sind zum Beispiel bekannt aus der WO 97/32880 und stellen wertvolle Arzneimittel dar, die insbesondere eine Hemmwirkung auf die durch Tyrosinkinasen vermittelte Signaltransduktion aufweisen.

Die dort beschriebene Herstellung ist jedoch für eine Produktion im technischen Maßstab ungeeignet, da sie die gewünschten Produkte zum Beispiel aus schwer zugänglichen Ausgangsmaterialien wie 4-Anilino-6-methylsulfinyl-pyrimido[5,4-*d*]pyrimidinen bzw. 4-Anilino-6-methylsulfonylpyrimido[5,4-*d*]pyrimidinen beschreibt, bei deren Umsetzung übelriechende und giftige Thiole freigesetzt werden, die zu einer Kontamination der Produkte führen.

Es ist daher Aufgabe der vorliegenden Erfindung, ein Verfahren bereitzustellen, das die großtechnisch durchführbare Synthese, Aufarbeitung, Reinigung und Isolierung von 4,6-Diaminopyrimido[5,4-*d*]pyrimidinen der Formel I erlaubt, bei der die vorstehend genannten Nachteile überwunden werden.

### Detaillierte Beschreibung der Erfindung

Überraschenderweise wurde gefunden, dass 4,6-Diaminopyrimido[5,4-*d*]pyrimidine der Formel I, worin
R¹ für ein Wasserstoffatom oder einen C₁-C₆ Alkylrest steht,
R² für einen gegebenenfalls substituierten C₆-C₁₀ Arylrest steht,
R³ für ein Wasserstoffatom oder einen C₁-C₆ Alkylrest steht, und
R⁴ für ein Wasserstoffatom oder einen gegebenenfalls substituierten C₁-C₆ Alkyl-, C₃-C₆ Alkenyl-, C₃-C₈ Cycloalkyl- oder einen 4- bis 7-gliedrigen, stickstoffhaltigen Heterocyclylrest steht, oder
R³ und R⁴ zusammen mit dem mit ihnen verknüpften Stickstoffatom für einen gegebenenfalls substituierten Heterocyclylrest stehen,
in hohen Ausbeuten und in einer vergleichsweise geringen Stufenzahl hergestellt werden können, indem man die Verbindung der Formel II, worin R¹, R², R³, und R⁴ die angegebene Bedeutung besitzen und
PG¹ für eine geeignete Schutzgruppe steht,
(a) nach Abspaltung der Schutzgruppe,
(b) mit einem Reduktionsmittel behandelt, und
(c) mit einem Oxidationsmittel behandelt.
Gegenstand der Erfindung ist somit ein Verfahren zur Herstellung der Verbindungen der Formel I.

Ein weiterer Gegenstand der Erfindung ist ein Verfahren zur Herstellung einer Verbindung der Formel II, indem man eine Verbindung der Formel III, 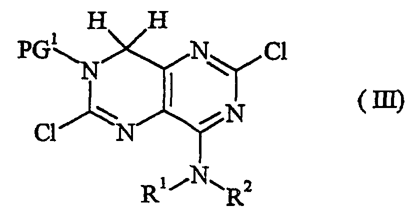 worin R¹, R² und PG¹ die angegebenen Bedeutungen besitzen,
mit einem Amin der Formel IV,

R³R⁴NH (IV)

worin R³ und R⁴ die angegebenen Bedeutungen besitzen,
gegebenenfalls in Gegenwart einer Base umsetzt.

Weiterhin Gegenstand der Erfindung ist ein Verfahren zur Herstellung einer Verbindung der Formel III, wobei man eine Verbindung der Formel V, 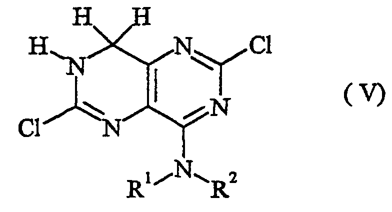 worin R¹ und R² die angegebene Bedeutung aufweisen, mit einer geeigneten Schutzgruppe (PG¹) versieht.

Ein weiterer Gegenstand der Erfindung ist ein Verfahren zur Herstellung der Verbindungen der Formel V, indem man eine Verbindung der Formel VI, 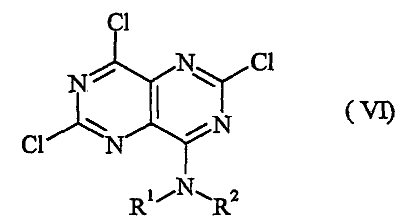 worin R¹ und R² die angegebene Bedeutung aufweisen, reduziert; sowie ein Verfahren zur Herstellung einer Verbindung der Formel VI, indem man 2,4,6,8- Tetrachlorpyrimido[5,4-*d*]pyrimidin mit einem Amin der Formel VIII,

R¹R²NH (VIII)

worin R¹ und R² die angegebene Bedeutung aufweisen, umsetzt.

Weiterhin Gegenstand der Erfindung sind die neuen Zwischenprodukte der Formeln II, III, V und VI.

Der Begriff "Alkyl" wie er vor- und nachstehend bezüglich der Gruppen R¹, R³ und/oder R⁴ verwendet wird, steht für eine geradkettige oder verzweigte Alkylgruppe mit bis zu 6 C-Atomen, vorzugsweise 1 bis 4 C-Atomen. Besonders bevorzugt sind Methyl, Ethyl, n-Propyl, *i*-Propyl, *n*-Butyl und *tert*-Butyl.

Der Begriff "Alkenyl" wie er vor- und nachstehend bezüglich der Gruppe R⁴ verwendet wird, steht für eine geradkettige oder verzweigte Alkenylgruppe mit bis zu 6 C-Atomen, vorzugsweise 3 bis 5 C-Atomen. Besonders bevorzugt sind Allyl, But-2-enyl, But-3-enyl, Pent-2-enyl, Pent-3-enyl und Pent-4-enyl.

Der Begriff "Aryl" wie er vor- und nachstehend bezüglich der Gruppe R² verwendet wird, steht für einen aromatischen Kohlenwasserstoffrest mit 6 bis 10 C-Atomen, vorzugsweise bedeutet Aryl Phenyl oder Naphthyl.

Der Begriff "geeignete Schutzgruppe" wie er vor- und nachstehend für die Gruppe PG¹ verwendet wird, steht für eine dem Fachmann geläufige Gruppe, z.B. eine in "Protective Groups in Organic Chemistry", Herausgeber J.F.W. McOmie (Plenum Press) beschriebene Schutzgruppen für Aminogruppen sein, die leicht eingeführt werden kann, gegenüber den nachfolgenden Reaktionen inert ist und leicht wieder abspaltbar ist. Bevorzugte Schutzgruppen sind die Acetyl-, Trifluoracetyl-, Benzoyl-, Ethoxycarbonyl-, *tert*-Butoxycarbonyl-, Benzyloxycarbonyl-, Benzyl-, Methoxybenzyl-, Mesyl-, Tosyl- oder 2,4-Dimethoxybenzylgruppe.

Die Abspaltung einer verwendeten Schutzgruppe erfolgt beispielsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z.B. in Gegenwart von Jodtrimethylsilan, bei Temperaturen zwischen 0 und 100°C, vorzugsweise bei Temperaturen zwischen 10 und 50°C.

Die Abspaltung eines Benzyl-, Methoxybenzyl- oder Benzyloxycarbonylrestes erfolgt beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0 und 50°C, vorzugsweise jedoch bei Raumtemperatur, erfolgen. Die Abspaltung eines 2,4-Dimethoxybenzylrestes erfolgt jedoch vorzugsweise in Trifluoressigsäure in Gegenwart von Anisol. Die Abspaltung eines *tert*-Butyloxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Der Begriff "Heterocyclylrest" wie er vor- und nachstehend bezüglich der Gruppe R³ bzw. der von R³ und R⁴ mit dem eingeschlossenen Stickstoffatom gebildeten Gruppe, verwendet wird, steht für einen gesättigten oder ungesättigten 4- bis 7-gliedrigen, stickstoffhaltigen Heterocyclylrest, welche neben Kohlenstoffatomen und mindestens einem Stickstoffatom gegebenenfalls weitere Heteroatome ausgewählt aus der Gruppe Sauerstoff und Schwefel aufweisen können. Dabei sind folgende Heterocyclylgruppen bevorzugt:
gesättigte 5- oder 6-gliedrige Heterocyclylgruppen, die
   - die ein oder zwei Stickstoffatome aufweisen, insbesondere Pyrrolidin, Piperidyl und Piperazyl, oder
   - ein Stickstoffatom und ein Sauerstoff- oder Schwefelatom aufweisen, insbesondere Morpholino,und Thiomorpholino.

Falls eine der Gruppen R¹ bis R⁴ als eine "gegebenenfalls substituierte" Gruppe bezeichnet ist, so kann diese Gruppe einen oder mehrere Substituenten, vorzugsweise 1, 2 oder 3, insbesondere 1 oder 2 Substituenten aufweisen, Diese Substituenten sind Gruppen, die unter den Reaktionsbedingungen des erfindungsgemäßen Verfahrens inert sind und keine nennenswerten Nebenreaktionen hervorrufen. Bevorzugte Substituenten sind ausgewählt aus der Gruppe bestehend aus Fluor, Chlor, Brom, Carboxy, Carboxy-C₁₋₃-Alkyl, Carboxy-C₁₋₃-alkoxy, Alkoxycarbonyl-C₁₋₃-alkoxy, Cyano, Trifluormethoxy, Trifluormethyl, C₁₋₃-Alkyl, Hydroxy, C₁₋₃-Alkoxy, Thiol, C₁₋₃-Alkylthio, Phenyl-C₁₋₃-alkoxy, Amino, Amino-C₁₋₃-Alkyl, C₁₋₃-Alkylamino C₁₋₃-Alkylamino-C₁₋₃-Alkyl, Di-(C₁₋₃-Alkyl)-amino und Di-(C₁₋₃-Alkyl)-amino-C₁₋₃-Alkyl.

In einer bevorzugten Ausführungsform weisen die Reste R¹ bis R⁴ folgende Bedeutungen auf
R¹ Wasserstoff oder Methyl, insbesondere Wasserstoff,
R² Phenyl, welches durch ein oder zwei Halogenatome, insbesondere Fluor und/oder Chlor substituiert sein kann, insbesondere 3-Chlor-4-fluorphenyl
R³ Wasserstoff oder Methyl, insbesondere Wasserstoff, und
R⁴ 5- oder 6-gliedrigen, stickstoffhaltigen Heterocyclus, welcher durch ein oder zwei C₁₋₃-Alkylreste substituiert sein kann, insbesondere 1-Methylpiperid-4-yl, oder
R³ und R⁴ bilden zusammen mit dem eingeschlossenen Stickstoffatom einen 5- oder 6-gliedrigen, stickstoffhaltigen Heterocyclus, welcher durch ein oder zwei Reste ausgewählt aus der Gruppe Amino, C₁₋₃-Alkyl und Di-(C₁₋₃-Alkyl)-amino-C₁₋₃-Alkyl, insbesondere Amino, Methyl und Diethylaminomethyl substituiert sein kann, meist bevorzugt 4-Amino-4-methylpiperid-1-yl.

In einer bevorzugten Ausführungsform des erfindungsgemäßen Verfahrens zur Herstellung der Verbindung der Formel I wird:
- in Schritt (b) die nach Abspaltung der Schutzgruppe erhaltene Verbindung in Gegenwart eines Übergangsmetallkatalysators hydriert oder mit Iodwasserstoff gegebenenfalls in Gegenwart von Phosphor behandelt;
- in Schritt (c) die in Schritt (b) erhaltene Verbindung mit einem Peroxodisulfat oder Wasserstoffperoxid behandelt.

### Stufe II → I

### Schritt (a)

Die Umsetzung der Verbindung der Formel II, worin PG¹ eine C₁₋₆ Alkylcarbonylgruppe ist, mit einer wässrigen Base, vorzugsweise einer Alkalibase wie Lithiumhydroxid, Natriumhydroxid oder Kaliumhydroxid, erfolgt in der Regel bei einer Temperatur von 0 °C bis 150 °C, vorzugsweise von 10 °C bis 100 °C, insbesondere bei etwa 40 bis 80 °C gegebenenfalls in Gegenwart eines inerten Lösungsmittels, wie zum Beispiel Toluol oder Tetrahydrofuran und eines protischen Lösungsmittels wie zum Beispiel eines Alkohols oder Wasser oder von Gemischen derselben. Vorzugsweise werden 0,8 bis 2, insbesondere 0,9 bis 1,2 Äquivalente der Base auf 1 Äquivalent der Verbindung der Formel II eingesetzt.

In einer besonders bevorzugten Ausführungsform werden etwa 0,9 bis 1,1 Äquivalente des Natriumhydroxids in Form einer etwa 2 normalen wässrigen Lösung bei Raumtemperatur zu einem Gemisch aus einem Äquivalent der Verbindung der Formel II und 5 bis 20 Teilen Methanol und 0,5 bis 2 Teilen Tetrahydrofuran (THF) bezogen auf 1 Teil Verbindung der Formel II gegeben und etwa 30 Minuten bis 2 Stunden zum Sieden erhitzt. Das erhaltene Gemisch wird nach Abkühlung ohne weitere Aufreinigung im nächsten Schritt (b) weiterverarbeitet.

### Schritt (b)

### Entchlorierung, Variante A:

Die nach Abspaltung der Schutzgruppe erhaltene Verbindung wird in der Regel in Gegenwart eines Übergangsmetallkatalysators hydriert. Die Hydrierung erfolgt in der Regel bei einer Temperatur von 20 °C bis 150 °C, vorzugsweise von 40 °C bis 120 °C, insbesondere bei der Siedetemperatur des Verdünnungsmittels, bei einem Wasserstoffdruck von etwa 1-120 bar in Gegenwart eines Ethers, wie zum Beispiel Diethylether, *tert*-Butyl-methylether, Dioxan oder Tetrahydrofuran, eines Alkohols, wie zum Beispiel Methanol, Ethanol oder Isopropanol, eines Kohlenwasserstoffes, wie zum Beispiel Cyclohexan oder n-Hexan oder von Gemischen derselben, insbesondere in Gegenwart des aus Schritt (a) vorliegenden Verdünnungsmittels. Anstelle des Wasserstoffs kann auch Ameisensäure als Wasserstoffdonator eingesetzt werden. In diesem Fall wird die Reaktion in der Regel bei Normaldruck und 5 bis 20 Teilen Ameisensäure auf 1 Teil des Katalysators eingesetzt. Vorzugsweise werden 0,01 bis 0,90 insbesondere 0,01 bis 0,10 Teile eines aus Palladium und Aktivkohle bestehenden Katalysators, der 1 bis 25 %, vorzugsweise 5 bis 15 % Palladium enthält, auf 1 Teil der nach Abspaltung der Schutzgruppe erhaltenen Verbindung eingesetzt. Die Hydrierung ist in der Regel unter den angegebenen Bedingungen nach 1 bis 100 Stunden, vorzugsweise 10 bis 80 Stunden beendet. Das erhaltene Rohprodukt wird nach Abdestillation des Lösungsmittels ohne weitere Aufreinigung im nächsten Schritt weiterverarbeitet.

In einer besonders bevorzugten Ausführungsform wird das in Schritt (a) erhaltene Gemisch zur Trockne eingeengt und der Rückstand in THF und Methanol aufgenommen. Es wird Pd/C (5-15 %-ig) hinzugefügt und zum Rückfluß erhitzt. Nach Zugabe von 2 bis 20 ml Ameisensäure wird 20 bis 75 Stunden gerührt. Man lässt auf Raumtemperatur erkalten, filtriert ab und engt zur Trockene ein. Der Rückstand wird mit Wasser und Essigester suspendiert, mit einer konzentrierten Ammoniaklösung schwach basisch gestellt und nachgerührt. Das kristalline Produkt wird isoliert, gegebenenfalls umkristallisiert und getrocknet.

### Entchlorierung, Variante B:

Alternativ kann die nach Abspaltung der Schutzgruppe erhaltene Verbindung mit Iodwasserstoff gegebenenfalls in Gegenwart von Phosphor behandelt werden. Die Umsetzung erfolgt in der Regel bei einer Temperatur von 20 °C bis 150 °C, vorzugsweise von 40 °C bis 120 °C, insbesondere bei der Siedetemperatur des Verdünnungsmittels, in Gegenwart eines polaren Lösungsmittels, insbesondere einer Carbonsäure, wie zum Beispiel Essigsäure oder Propionsäure, oder einer wässrigen Mineralsäure wie zum Beispiel Salzsäure, Bromwasserstoffsäure, Iodwasserstoffsäure oder von Gemischen derselben oder Wasser. Die Reaktion wird in der Regel bei Normaldruck durchgeführt, wobei vorzugsweise 6 bis 6,5 Äquivalente Iodwasserstoff in Form einer 30- bis 60 %-igen wässrigen Lösung bezogen auf 1 Äquivalent der nach Abspaltung der Schutzgruppe erhaltenen Verbindung eingesetzt werden. Vorzugsweise wird die Reaktion in Gegenwart von rotem Phosphor durchgeführt, wobei 1,0 bis 3,0, vorzugsweise 1,8 bis 2,5 Äquivalente Phosphor bezogen auf 1 Äquivalent der nach Abspaltung der Schutzgruppe erhaltenen Verbindung eingesetzt werden. Die Umsetzung ist in der Regel unter den angegebenen Bedingungen nach 0,5 bis 10 Stunden, vorzugsweise 1 bis 8 Stunden beendet. Das erhaltene Rohprodukt wird nach Abdestillation des Lösungsmittels ohne weitere Aufreinigung im nächsten Schritt weiterverarbeitet.

In einer besonders bevorzugten Ausführungsform wird das in Schritt (a) erhaltene Gemisch eingeengt, in Eisessig aufgenommen und in eine Suspension von rotem Phosphor in Iodwasserstoffsäure (57 %-ig) getropft. Die Mischung wird 3 bis 5 Stunden unter Rückfluß erhitzt, entfärbt und neutralisiert. Das Produkt wird isoliert und getrocknet.

### Schritt (c)

Die in Schritt (b) erhaltene Verbindung wird vorzugsweise in Gegenwart einer wässrigen Säure mit einem Peroxodisulfat oder Wasserstoffperoxid oxidiert. Die Oxidation erfolgt in der Regel bei einer Temperatur von 0 °C bis 150 °C, vorzugsweise von 40 °C bis 120 °C, insbesondere bei der Siedetemperatur des Verdünnungsmittels, in Gegenwart einer Säure, wie zum Beispiel Essigsäure, Trifluoressigsäure, Salzsäure, Salpetersäure, Phosphorsäure oder Schwefelsäure oder von Gemischen derselben, insbesondere in Gegenwart von Wasser. Vorzugsweise werden 0,8 bis 2,0. insbesondere 0,9 bis 1,5 Äquivalente der Oxidationsmittels, insbesondere in Form einer 20- bis 40 %-igen wässrigen Lösung von Wasserstoffperoxid oder einer 20- bis 50 %-igen wässrigen Lösung von Natriumperoxodisulfat bezogen auf Äquivalent der in Schritt (b) erhaltenen Verbindung. Die Oxidation ist in der Regel unter den angegebenen Bedingungen nach 10 Minuten bis 30 Stunden, vorzugsweise 15 Minuten bis 24 Stunden beendet. Das erhaltene Rohprodukt wird nach Abdestillation des Lösungsmittels ohne weitere Aufreinigung im nächsten Schritt weiterverarbeitet.

In einer besonders bevorzugten Ausführungsform wird 1 Äquivalent der in Schritt (b) erhaltenen Verbindung in 5- bis 15 %-iger Schwefelsäure aufgenommen und in der Siedehitze mit 0,01 bis 0,-10 Äquivalenten Kaliumiodid und 1,0 bis 2,0, vorzugsweise 1,2 bis 1,5 Äquivalenten Wasserstoffperoxid in Form einer 35 %-igen Lösung gegeben. Man lässt 10 bis 30 Minuten rühren, gibt eine wässrige Lösung von Natriumbisulfit sowie Ethanol zu. Anschließend lässt man langsam auf Raumtemperatur abkühlen und rührt nach. Das auskristallisierte Produkt wird abfiltriert und getrocknet.

In einer weiteren besonders bevorzugten Ausführungsform wird 1 Äquivalent der in Schritt (b) erhaltenen Verbindung in einem Gemisch aus Eisessig und Wasser (20- bis 40 %-ig) aufgenommen und mit 1,0 bis 2,0, vorzugsweise 1,1 bis 1,6 Äquivalenten von Natriumperoxodisulfat in Form einer etwa 30- bis 35 %-igen wässrigen Lösung versetzt. Man rührt 30 bis 300 Minuten bei einer Temperatur von 20 bis 75 °C. Der entstandene Feststoff wird abfiltriert, in Wasser suspendiert, mit konzentrierter Ammoniaklösung neutralisiert und mit Essigester extrahiert. Die organische Phase wird abgetrennt, getrocknet und eingeengt. Der Rückstand wird in Ethanol aufgenommen und durch Zugabe von ethanolischer HCl und in das Hydrochlorid der Verbindung der Formel I überführt. Das kristallisierte Produkt abfiltriert und getrocknet.

### Stufe III → II

In einer bevorzugten Ausführungsform des erfindungsgemäßen Verfahrens zur Herstellung der Verbindung der Formel II wird:
- die Reaktion in Gegenwart eines tertiären Amins durchgeführt;
- die Umsetzung in einem Temperaturbereich von 0°C bis 150°C durchgeführt.

Die Umsetzung mit dem Amin der Formel IV wird zweckmäßigerweise in einem Verdünnungsmittel wie Methanol, Ethanol, Isopropanol, Butanol, Tetrahydrofuran, Dioxan, Toluol, Chlorbenzol, Dimethylformamid, Dimethylsulfoxid, Ethylenglykolmonomethylether, Ethylenglycoldiethylether oder Sulfolan oder Gemischen dieser Verdünnungsmittel, gegebenenfalls in Gegenwart einer anorganischen Base, z.B. Natriumcarbonat oder Kaliumhydroxid, oder einer tertiären organischen Base, z.B. Triethylamin, N-Ethyl-diisopropylamin oder Pyridin, wobei letztere gleichzeitig auch als Lösungsmittel dienen können, und gegebenenfalls in Gegenwart eines Reaktionsbeschleunigers wie eines Kupfersalzes, eines entsprechenden Amin-hydrohalogenids oder Alkalihalogenids bei Temperaturen zwischen 0 und 150°C, vorzugsweise jedoch bei Temperaturen zwischen 20 und 120°C, durchgeführt. Die Umsetzung kann jedoch auch ohne Lösungsmittel oder in einem Überschuss der eingesetzten Verbindung der allgemeinen Formel IV durchgeführt werden. Die Reaktion wird in der Regel bei Normaldruck durchgeführt, wobei 0,8 bis 2,0, vorzugsweise 0,9 bis 1,5, insbesondere 1,0 bis 1,5 Äquivalente einer Verbindung der Formel IV bezogen auf 1 Äquivalent der Verbindung der Formel III eingesetzt werden. Die Umsetzung ist in der Regel unter den angegebenen Bedingungen nach 15 bis 600 Minuten, vorzugsweise 30 bis 180 Minuten beendet. Das erhaltene Rohprodukt wird in der Regel durch Umkristallisation gereinigt oder kann ohne weitere Aufreinigung im nächsten Schritt weiterverarbeitet.

In einer besonders bevorzugten Ausführungsform wird zu einem Gemisch aus 1 Äquivalent der Verbindung der Formel III, THF und/oder Methanol und gegebenenfalls 1 bis 2 Äquivalenten Triethylamin bei Raumtemperatur 0,8 bis 1,8, vorzugsweise 1,0 bis 1,6 Äquivalente des Amins der Formel IV, insbesondere 1-Methyl-4-aminopiperidin oder 4-Methylpiperidin-4-ylamin getropft. Die Mischung wird 10 bis 90 Minuten bei 20 bis 80 °C gerührt. Nach Abkühlen, gibt man Wasser hinzu und rührt nach. Das auskristallisierte Produkt wird abfiltriert, mit Wasser gewaschen und getrocknet. Nach Umkristallisation aus Essigester wird filtriert und getrocknet.

### Stufe V → III

In einer bevorzugten Ausführungsform des erfindungsgemäßen Verfahrens zur Herstellung der Verbindung der Formel III wird:
- die Verbindung der Formel V mit einer C₁₋₆ Carbonsäure oder einem reaktiven Derivat davon in Gegenwart einer Base, vorzugsweise einem C₁₋₆ Carbonsäurechlorid in Gegenwart eines tertiären Amins, insbesondere Acetylchlorid in Gegenwart von N-Ethyldiisopropylamin umgesetzt.

Die Acylierung wird zweckmäßigerweise mit einem entsprechenden Halogenid oder Anhydrid in einem Lösungsmittel wie Methylenchlorid, Chloroform, Tetrachlorkohlenstoff, Ether, Tetrahydrofuran, Essigsäureethylester, Dioxan, Benzol, Toluol, Acetonitril oder Sulfolan gegebenenfalls in Gegenwart einer anorganischen oder organischen Base, vorzugsweise einem tertiären Amin bei Temperaturen von -20 bis 150°C, vorzugsweise bei Temperaturen von -10 bis 120°C, insbesondere bei Raumtemperatur durchgeführt. Diese kann jedoch auch mit der freien Säure gegebenenfalls in Gegenwart eines die Säure aktivierenden Mittels oder eines wasserentziehenden Mittels, z.B. in Gegenwart von Chlorameisensäureisobutylester, Thionylchlorid, Trimethylchlorsilan, Chlorwasserstoff, Schwefelsäure, Methansulfonsäure, p-Toluolsulfonsäure, Phosphortrichlorid, Phosphorpentoxid, *N,N'*-Dicyclohexylcarbodiimid, *N,N'*-Dicyclohexylcarbodiimid / *N*-Hydroxysuccinimid oder 1-Hydroxybenzotriazol, *N,N'*-Carbonyldiimidazol oder *N,N'*-Thionyldiimidazol oder Triphenylphosphin/Tetrachlorkohlenstoff, bei Temperaturen zwischen -20 und 200°C, vorzugsweise jedoch bei Temperaturen zwischen -10 und 160°C, durchgeführt werden. Die Reaktion wird in der Regel bei Normaldruck durchgeführt, wobei 0,8 bis 2,0, vorzugsweise 0,9 bis 1,5, insbesondere 1,0 bis 1,5 Äquivalente eines Carbonsäurederivates bezogen auf 1 Äquivalent der Verbindung der Formel V eingesetzt werden. Die Umsetzung ist in der Regel unter den angegebenen Bedingungen nach 15 bis 300 Minuten, vorzugsweise 30 bis 120 Minuten beendet. Das erhaltene Rohprodukt wird in der Regel durch Umkristallisation gereinigt oder kann ohne weitere Aufreinigung im nächsten Schritt weiterverarbeitet.

In einer besonders bevorzugten Ausführungsform werden zu einem Gemisch aus 1 Äquivalent der Formel V, *N*-Ethyldiisopropylamin und Essigester bei 0 bis 20 °C 1,1 bis 1,5, insbesondere etwa 1,3 Äquivalente Acetylchlorid getropft. Man rührt 60 bis 120 Minuten bei Raumtemperatur und wäscht mit Wasser bei 40 bis 90 °C. Das Produkt wird aus Ethanol umkristallisiert, abfiltriert und getrocknet.

### Stufe VI → V

In einer bevorzugten Ausführungsform des erfindungsgemäßen Verfahrens zur Herstellung der Verbindung der Formel V wird:
- die Verbindung der Formel VI mit einem Boranat, insbesondere mit Natriumboranat in Gegenwart einer Base und eines polaren Lösungsmittels reduziert.

Die Reduktion kann mittels katalytischer Hydrierung mit Wasserstoff in Gegenwart eines Katalysators wie Palladium/Kohle oder Platin in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar durchgeführt werden.

Vorzugsweise erfolgt die Reduktion mit einem Boranat ausgewählt aus der Gruppe Lithiumboranat, Natriumboranat, Kaliumboranat zweckmäßigerweise in einem Verdünnungsmittel wie Wasser, Methanol, Ethanol, Isopropanol, Butanol, Tetrahydrofuran, Dioxan, Toluol, Chlorbenzol, Dimethylformamid, Dimethylsulfoxid, Ethylenglykolmonomethylether, Ethylenglycoldiethylether oder Sulfolan oder Gemischen dieser Verdünnungsmittel, gegebenenfalls in Gegenwart einer anorganischen Base, z.B. Natriumcarbonat, Natriumhydroxid oder Kaliumhydroxid, oder einer tertiären organischen Base, z.B. Triethylamin, *N*-Ethyldiisopropylamin oder Pyridin, bei Temperaturen von - 20 bis + 60 °C, vorzugsweise -10 bis + 20 °C. Die Reaktion wird in der Regel bei Normaldruck durchgeführt, wobei 0,9 bis 4,0, vorzugsweise 1,1 bis 1,9, insbesondere 1,6 bis 1,8 Mol eines Boranats bezogen auf 1 Mol der Verbindung der Formel VI eingesetzt werden. Die Umsetzung ist in der Regel unter den angegebenen Bedingungen nach 15 bis 600 Minuten, vorzugsweise 60 bis 240 Minuten beendet. Das erhaltene Rohprodukt wird in der Regel durch Umkristallisation gereinigt oder kann ohne weitere Aufreinigung im nächsten Schritt weiterverarbeitet.

In einer besonders bevorzugten Ausführungsform wird zu einem Gemisch aus 1 Mol einer Verbindung der Formel VI und Essigester bei 0 bis 5 °C ein Gemisch von etwa 1,75 Mol Natriumborhydrid, Wasser und Natriumhydroxid in Form einer etwa 2N Natronlauge getropft. Man lässt 1 bis 3 Stunden bei 0 bis 20 °C nachrühren und erwärmt dann auf 60 bis 80 °C. Die Suspension wird filtriert und auf etwa 0 °C gekühlt. Das auskristallisierte Produkt wird isoliert und getrocknet.

### Stufe Tetrachlorhomopurin → VI

Die Umsetzung mit dem Amin der Formel VII wird zweckmäßigerweise in einem Verdünnungsmittel wie Methanol, Ethanol, Isopropanol, Butanol, Tetrahydrofuran, Dioxan, Toluol, Chlorbenzol, Dimethylformamid, Dimethylsulfoxid, Ethylenglykolmonomethylether, Ethylenglycoldiethylether, Sulfolan oder Wasser oder Gemischen dieser Verdünnungsmittel, insbesondere THF, gegebenenfalls in Gegenwart einer anorganischen Base, z.B. Natriumcarbonat oder Kaliumhydroxid, oder einer tertiären organischen Base, z.B. Triethylamin, *N*-Ethyldiisopropylamin oder Pyridin, bei Temperaturen von 0 bis 80°C, vorzugsweise 5 bis 50 °C, insbesondere 10 bis 30 °C durchgeführt. Die Reaktion wird in der Regel bei Normaldruck durchgeführt, wobei 0,7 bis 1,2, vorzugsweise 0,8 bis 1,0 insbesondere etwa 0,95 Äquivalente einer Verbindung der Formel VII bezogen auf 1 Äquivalent wasserfeuchtes Tetrachlorohomopurin eingesetzt werden. Die Umsetzung ist in der Regel unter den angegebenen Bedingungen nach 15 bis 600 Minuten, vorzugsweise 30 bis 180 Minuten beendet. Das erhaltene Rohprodukt wird in der Regel durch Umkristallisation gereinigt oder kann ohne weitere Aufreinigung im nächsten Schritt weiterverarbeitet.

In einer besonders bevorzugten Ausführungsform wird zu einem Gemisch aus 1 Äquivalent Tetrachlorhomopurin und Tetrahydrofuran bei 10 bis 20 °C eine Lösung von 0,8 bis 0,95 Äquivalenten des Amins der Formel VII, insbesondere 3-Chlor-4-fluoranilin in THF getropft. Man rührt 10 bis 50 Minuten bei Raumtemperatur, fügt Aktivkohle hinzu und rührt weitere 10 bis 20 Minuten bei Raumtemperatur. Anschließend wird das Reaktionsgemisch in Wasser filtriert, der Filterrückstand mit THF nachgewaschen. Die wässrige Suspension wird bei Raumtemperatur gerührt und das Produkt anschließend isoliert und getrocknet.

Ein wesentlicher Vorteil des erfindungsgemäßen Verfahrens ist, dass es die technische Herstellung der Verbindungen der Formel I ausgehend von einem leicht zugänglichen Ausgangsmaterial, nämlich 2,4,6,8-Tetrachlorpyrimido[5,4-*d*]pyrimidin (Tetrachlorhomopurin) ermöglicht.

Weitere vorteilhafte Aspekte der erfindungsgemäßen Vorgehensweise sind die hohe Raum-Zeit-Ausbeute beim vorliegenden Prozess sowie die hohe Ausbeute und Reinheit an den jeweiligen Zwischenprodukten, welche ohne chromatographische Aufreinigung weiterverarbeitet werden können.

Die nachfolgenden Beispiele dienen der Illustration exemplarisch durchgeführter Verfahren zur Herstellung der Verbindung der Formel I. Sie sind lediglich als mögliche, exemplarisch dargestellte Vorgehensweisen zu verstehen, ohne die Erfindung auf deren Inhalt zu beschränken.

### Beispiel 1

### (3-Chlor-4-fluorphenyl)-(2,6,8-trichlorpyrimido[5,4-d]pyrimidin-4-yl)amin (1)

In eine Suspension von 269,3 g wasserfeuchtem Tetrachlorhomopurin (ca. 50%ig, entspr. ca. 0,5mol) in 1820 ml Tetrahydrofuran (THF) wird bei 15 °C eine Lösung von 72,78 g (0,5 mol) 3-Chlor-4-fluoranilin in 200 ml THF getropft. Man rührt 30 Minuten bei Raumtemperatur, fügt 13,5 g Aktivkohle hinzu und rührt weitere 15 Minuten bei Raumtemperatur. Anschließend wird das Reaktionsgemisch in 4,11 Wasser filtriert, der Filterrückstand mit 200 ml THF nachgewaschen. Die wässrige Suspension wird 30 Minuten bei Raumtemperatur gerührt und das Produkt anschließend abfiltriert und im Vakuum getrocknet. Ausbeute: 189 g (99,7 %, bezogen auf das Anilin), Schmp. 229,.6°C. 250MHz-¹H-NMR (CDCl₃) δ (ppm) = 8,83 br s, 1H, NH; 7,95 dd, 1H, Anilin-H; 7,69 ddd, 1H, Anilin-H; 7,19 dd, 1H, Anilin-H.

### Beispiel 2

### (3-Chlor-4-fluorphenyl)-(2,6-dichlor-7,8-dihydropyrimido[5,4-d]pyrimidin-4-yl)amine (2)

In eine Suspension von 1100 g (2,902 mol) **(1)** in 22 1 Essigester wird bei 2 °C eine Lösung von 191,99 g (5,079 mol) Natriumborhydrid in 520 ml Wasser und 22 ml 2N Natronlauge getropft. Man lässt 2 Stunden bei 10 °C nachrühren und erwärmt dann auf 71 °C. Die Suspension wird über Celite filtriert und auf 0 °C gekühlt. Das auskristallisierte Produkt wird abfiltriert und bei 40 °C bis zur Gewichtskonstanz getrocknet. Ausbeute: 957,1 g (95,2 %). Schmp.: 227,4 °C.
250-MHz-¹H-NMR (DMSO) δ (ppm) = 9,15 br s, 1H, NH; 8,79 br s, 1H, NH; 8,00, dd, 1H, Anilin-H; 7,73 ddd, 1H, Anilin-H; 7,39 t, 1H, Anilin-H; 4,55 s, 2H, CH₂.

### Beispiel 3

### 1-[2,6-Dichlor-8-(3-chlor-4-fluorphenylamino)-4H-pyrimido[5,4-d]pyrimidin-3-yl]ethanon (3)

In eine Suspension von 210,6 g (0,608 mol) **(2)** und 176ml *N*-Ethyldiisopropylamin (0,95 mol) in 2.,1 l Essigester werden bei 10 °C 56,4 ml (0,789 mol) Acetylchlorid getropft. Man rührt 90 Minuten bei Raumtemperatur und wäscht anschließend 3 mal mit je 700 ml Wasser bei 60 °C. Die organische Phase wird eingeengt und mit 590 ml Ethanol versetzt. Man lässt kurz aufkochen, auf 0-5 °C abkühlen und rührt bei dieser Temperatur 30 Minuten nach. Das auskristallisierte Produkt wird abfiltriert und bei 60 °C im Vakuum getrocknet. Ausbeute: 180,3 g (76,3 %). Schmp.: 177,6 °C.
250MHz-¹H-NMR (CDCl₃) δ (ppm) = 7,80 dd, 1H, Anillin-H; 7,70 s, 1H, NH; 7,55 ddd, 1H, Anilin-H; 7,15 t, 1H, Anilin-H; 4,89 s, 2H, CH₂; 2,51 s, 3H, CH₃.

### Beispiel 4

### 1-[6-Chlor-8-(3-chlor-4-fluorphenylamino)-2-(1-methylpiperidin-4-ylamino)-4Hpyrimido[5,4-d]pyrimidin-3-yl]ethanon (4)

In ein Gemisch aus 123,3 g (0,317 mol) **(3)**, 740 ml THF und 61,7 ml (0,445 mol) Triethylamin werden bei Raumtemperatur 47,2 g (0,413 mol) 1-Methyl-4-aminopiperidin getropft. Die Mischung wird 30 min bei 50 °C gerührt. Man lässt auf 40 °C abkühlen, gibt 1480 ml Wasser hinzu und rührt 90 Minuten bei 10 °C. Das auskristallisierte Produkt wird abfiltriert, mit 150 ml Wasser gewaschen und im Vakuum bei 60 °C getrocknet. Das Produkt wird in 1150 ml Essigester in der Siedehitze gelöst. Man kühlt auf 10 °C ab, filtriert und trocknet das auskristallisierte Produkt bei 60 °C im Vakuum. Ausbeute: 128,9 g (87,2 %).
250-MHz-¹H-NMR (CDCl₃) δ (ppm) = 8,70 br s, 1H, NH; 7.80 dd, 1H, Anilin-H; 7,72 s, 1H, NH; 7,50 ddd, 1H, Anilin-H; 7.13 t, 1H, Anilin-H; 4,75 s, 2H, CH₂; 4,03-3,87 m, 1H; CH₂CHNCH₂; 2,82-2,65 m, 2H, CH₂CH₂N; 2,38 s, 3H, CH₃; 2,33 s, 3H, CH₃; 2,33-2,19 m, 2H, CH₂CH₂N; 2,18-2,02 m, 2H, CHCH₂CH₂; 1,75-1,60 m, 2H, CHCH₂CH₂.

### Beispiel 5

### 8-(3-Chlor-4-fluorphenylamino)-2-(1-methylpiperidin-4-ylamino)pyrimido[5,4-d]pyrimidin (5)

In ein Gemisch aus 9 g (0,019 mol) **(4)**, 81 ml Methanol und 9 ml THF werden 9 ml 2N Natronlauge gegeben und die Mischung 1 Stunde unter Rückfluß erhitzt. Dann werden 0,45 g Pd/C (10 %-ig) zugefügt und in der Siedehitze 4,5 ml Ameisensäure zugetropft.

Man lässt 24 Stunden unter Rückfluß sieden, filtriert ab und engt im Vakuum zum Rückstand ein. Dieser wird in 90 ml Wasser und 7,2 ml konz. Bromwasserstoffsäure aufgenommen und bei Raumtemperatur mit 3,9 ml Wasserstoffperoxid (30 %-ig) versetzt. Man lässt 5 Stunden bei Raumtemperatur rühren, filtriert anschließend das auskristallisierte Produkt ab und wäscht mit Wasser nach. Der Filterrückstand wird in Methanol gelöst, die freie Base durch Zugabe von 30 ml Wasser und 2N Natronlauge ausgefällt. Ausbeute: 4,95 g (66 %)

### Beispiel 6

### [6-(4-Amino-4-methylpiperidin-1-yl)-2-chlor-7,8-dihydropyrimido [5,4-d]pyrimidin-4-yl]-(3-chlor-4-fluorphenyl)amin (6)

In eine Suspension von 100 g (0,257 mol) **(3)** in 690 ml Methanol wird bei 60 °C ein Gemisch von 30,8 g (0,270mol) 4-Methylpiperidin-4-ylamin und 100 ml Methanol getropft. Man rührt 1 Stunde bei 60 °C, lässt auf 40 °C erkalten, gibt 280 ml 2N Natronlauge zu und rührt 3 Stunden bei 60 °C. Anschließend werden 300 ml Methanol abdestilliert und 600 ml Wasser zugegeben. Man lässt 5 Minuten bei Raumtemperatur. rühren, filtriert ab und wäscht den Rückstand mit 250 ml Wasser, 3 mal 50 ml Aceton, 100 ml Methyl-*tert*-butylether nach und trocknet bei 40 °C im Vakuum. Ausbeute 100,5 g (92,2 %). Schmp. 211,5 °C.
400MHz-¹H-NMR (DMSO) δ (ppm) = 8,55 br s, 1H, NH; 8,05 dd, 1H, Anilin-H; 7,78 ddd, 1H, Anilin-H; 7,47 t, 1H, Anilin-H; 6,90 br s, 1H, NH; 4,28 s, 2H, CH₂; 3,68 m, 2H, CH₂; 3,45 m, 2H, CH₂, 3,32 br s, 2H, NH₂; 1,37 m, 4H, 2CH₂; 1,05 s, 3H, CH₃.

### Beispiel 7

### [6-(4-Amino-4-methylpiperidin-1-yl)-4-(3-chlor-4-fluorphenylamino)-pyrimido[5,4-d]pyrimidin (7)

### Entchlorierung:

In eine Suspension von 11,7 g (0,378 mol) rotem Phosphor in 150 ml Iodwasserstoffsäure (57 %-ig, 1,134 mol) wird ein 90 °C warmes Gemisch von 80 g (0,182 mol) **(6)** in 160 ml Eisessig getropft. Man erhitzt 4 Stunden unter Rückfluß, gibt 5 g Aktivkohle zu, filtriert ab und wäscht den Rückstand mit Eisessig und Wasser nach. Das Filtrat wird unter kräftigem Rühren in eine Lösung von 270 ml Wasser, 270 ml Methanol und 270 ml Natronlauge (50 %-ig) getropft. Man lässt bei 15 °C 30 Minuten nachrühren und filtriert den ausgefallenen Feststoff ab. Dieser wird 2 mal mit 500 ml Wasser gewaschen, anschließend in 500 ml Wasser suspendiert, abfiltriert und bei 45 °C im Vakuum getrocknet. Ausbeute: 65,2 g (91,9 %)

### Oxidation, Variante A:

In eine Lösung von 34 g (0,084 mol) der entchlorierten Verbindung in 500 ml Wasser und 38,7 ml konzentrierter Schwefelsäure werden in der Siedehitze 0,35 g (0,002 mol) Kaliumiodid und 10,9 ml (0,112 mol) 35 %-iges Wasserstoffperoxid gegeben. Man lässt 20 Minuten rühren, gibt eine Lösung von 7 g Natriumbisulfit in 10ml Wasser sowie 150 ml Ethanol zu. Anschließend lässt man langsam auf Raumtemperatur abkühlen und rührt 3 Stunden nach. Das auskristallisierte Produkt wird abfiltriert und im Vakuum bei 40 °C getrocknet. Ausbeute 34,3 g (84,1 %) in Form des Sulfats.
Eine Suspension von 42 g des Sulfats in 600 ml Wasser und 40 ml konzentrierter Ammoniaklösung wird mit 1,5 1 Essigester extrahiert. Die vereinigten organischen Phasen werden mit Wasser gewaschen, mit Natriumsulfat getrocknet und im Vakuum bis zur beginnenden Kristallisation eingeengt. Man lässt 2 Stunden bei 0 °C rühren, filtriert das auskristallisierte Produkt ab und trocknet im Vakuum. Ausbeute: 25,5 g (76,6 %).

### Oxidation, Variante B:

In eine Lösung von 10 g (0,025 mol) der entchlorierten Verbindung in 100 ml Wasser und 5,7 ml Eisessig wird bei Raumtemperatur eine Lösung von 8,9 g (0,037 mol) Natriumperoxodisulfat in 25 ml Wasser getropft. Man rührt 30 Minuten bei 60 °C und über Nacht bei Raumtemperatur. Danach werden 200 ml Essigester und 30 ml konzentrierte Ammoniaklösung zugegeben. Die Phasen werden getrennt, die wässrige Phase mit 100 ml Essigester extrahiert und die vereinigten organischen Phasen zur Trockne eingeengt. Der Rückstand wird in 140 ml Essigester in der Siedehitze aufgenommen, dann werden 80 ml Essigester abdestilliert und der Rückstand auf 0 °C gekühlt. Das auskristallisierte Produkt wird abfiltriert und im Vakuum getrocknet. Ausbeute: 6,2 g (63,9 %).

## Patentansprüche

1. Verfahren zur Herstellung von 4,6-Diaminopyrimido[5,4-*d*]pyrimidinen der Formel I, 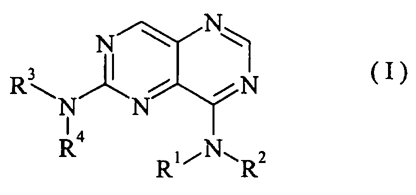 wobei
R¹ für ein Wasserstoffatom oder einen C₁-C₆ Alkylrest steht,
R² für einen gegebenenfalls substituierten C₆-C₁₀ Arylrest steht,
R³ für ein Wasserstoffatom oder einen C₁-C₆ Alkylrest steht, und
R⁴ für ein Wasserstoffatom oder einen gegebenenfalls substituierten C₁-C₆ Alkyl-, C₃-C₆ Alkenyl-, C₃-C₈ Cycloalkyl- oder einen 4- bis 7-gliedrigen, stickstoffhaltigen Heterocyclylrest steht, oder
R³ und R⁴ zusammen mit dem mit ihnen verknüpften Stickstoffatom für einen gegebenenfalls substituierten Heterocyclylrest stehen,
**dadurch gekennzeichnet, dass** man die Verbindung der Formel II, 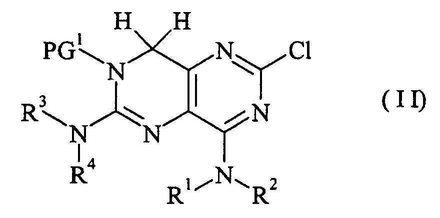 worin R¹, R², R³, und R⁴ die angegebene Bedeutung besitzen und
PG¹ für eine geeignete Schutzgruppe steht,
(a) nach Abspaltung der Schutzgruppe,
(b) mit einem Reduktionsmittel behandelt, und
(c) mit einem Oxidationsmittel behandelt.

2. Verfahren nach Anspruch 1, **dadurch gekennzeichnet, dass** die Schutzgruppe eine C₁₋₆ Alkylcarbonylgruppe ist und man diese durch Behandeln mit einer wässrigen Base abspaltet.

3. Verfahren nach Anspruch 1 oder 2, **dadurch gekennzeichnet, dass** man in Schritt (b) die nach Abspaltung der Schutzgruppe erhaltene Verbindung in Gegenwart eines Übergangsmetallkatalysators hydriert oder mit Iodwasserstoff gegebenenfalls in Gegenwart von Phosphor behandelt.

4. Verfahren nach einem der Ansprüche 1 bis 3, **dadurch gekennzeichnet, dass** man in Schritt (c) die in Schritt (b) erhaltene Verbindung mit einem Peroxodisulfat oder Wasserstoffperoxid behandelt.

5. Verfahren zur Herstellung einer Verbindung der Formel II gemäß Anspruch 1, **dadurch gekennzeichnet, dass** man eine Verbindung der Formel III, 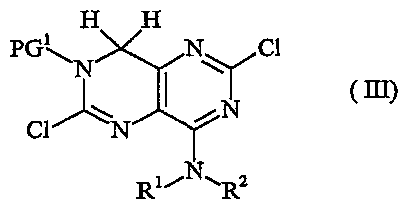 worin R¹, R² und PG¹ die angegebenen Bedeutungen besitzen,
mit einem Amin der Formel IV,
R³R⁴NH (IV)
worin R³ und R⁴ die angegebenen Bedeutungen besitzen,
gegebenenfalls in Gegenwart einer Base umsetzt.

6. Verfahren nach Anspruch 5, **dadurch gekennzeichnet, dass** man die Reaktion in Gegenwart eines tertiären Amins durchführt.

7. Verfahren nach Anspruch 5 oder 6, **dadurch gekennzeichnet, dass** die Umsetzung in einem Temperaturbereich von 0°C bis 150°C durchgeführt wird.

8. Verfahren zur Herstellung einer Verbindung der Formel III gemäß Anspruch 5, **dadurch gekennzeichnet, dass** man eine Verbindung der Formel V, 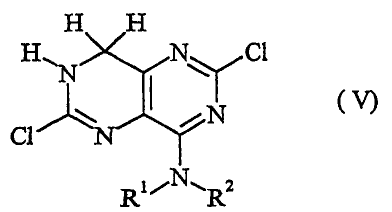 worin R¹ und R² die angegebene Bedeutung aufweisen, mit einer geeigneten Schutzgruppe (PG¹) versieht.

9. Verfahren nach Anspruch 8, **dadurch gekennzeichnet, dass** man die Verbindung der Formel V mit einer C₁₋₆ Carbonsäure oder eines reaktiven Derivates davon in Gegenwart einer Base umsetzt.

10. Verfahren nach Anspruch 8 oder 9, **dadurch gekennzeichnet, dass** man die Verbindung der Formel V mit einem C₁₋₆ Carbonsäurechlorid in Gegenwart eines tertiären Amins umsetzt.

11. Verfahren zur Herstellung einer Verbindung der Formel V gemäß Anspruch 8, **dadurch gekennzeichnet, dass** man eine Verbindung der Formel VI, 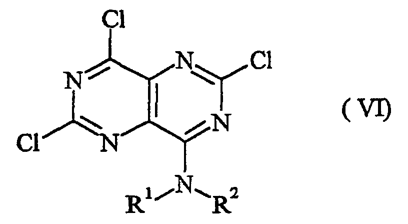 worin R¹ und R² die angegebene Bedeutung aufweisen, reduziert.

12. Verfahren nach Anspruch 11, **dadurch gekennzeichnet, dass** man die durch Reduktion mit einem Boranat durchführt.

13. Verfahren nach Anspruch 12, **dadurch gekennzeichnet, dass** man die Reduktion mit Natriumboranat in Gegenwart einer Base und eines polaren Lösungsmittels durchführt.

14. Verfahren zur Herstellung einer Verbindung der Formel VI gemäß Anspruch 11, **dadurch gekennzeichnet, dass** man 2,4,6,8-_Tetrachlorpyrimido[5,4-*d*]pyrimidin mit einem Amin der Formel VIII,
R¹R²NH (VIII)
worin R¹ und R² die angegebene Bedeutung aufweisen, umsetzt.

15. 2-Chlor-4,6-diamino-7,8-dihydropyrimido[5,4-*d*]pyrimidin der Formel II 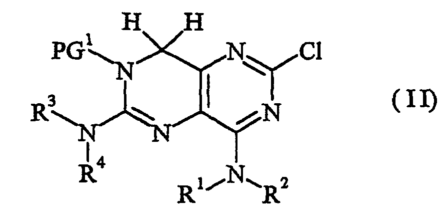 worin R¹, R², R³, und R⁴ die angegebene Bedeutung besitzen und
PG¹ für eine C₁₋₆ Alkanoylgruppe steht.

16. 4-Amino-2,6-dichlor-7,8-dihydropyrimido[5,4-*d*]pyrimidin der Formel III 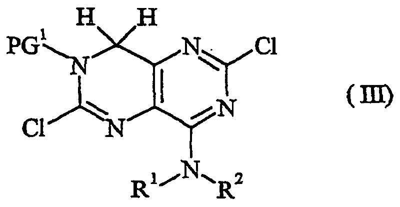 worin R¹ und R² die angegebenen Bedeutungen besitzen, und
PG¹ für eine C₁₋₆ Alkanoylgruppe steht.

17. 4-Amino-2,6-dichlor-7,8-dihydropyrimido[5,4-*d*]pyrimidin der Formel V, 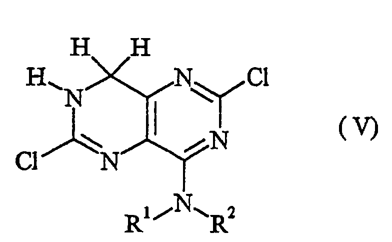 worin R¹ und R² die angegebene Bedeutung aufweisen.

18. 4-Amino-2,6,8-trichlorpyrimido[5,4-*d*]pyrimidin der Formel VI, 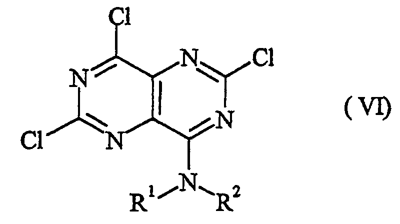 worin R¹ und R² die angegebene Bedeutung aufweisen.

## Claims

1. Process for preparing 4,6-diaminopyrimido[5,4-*d*]pyrimidines of formula I, 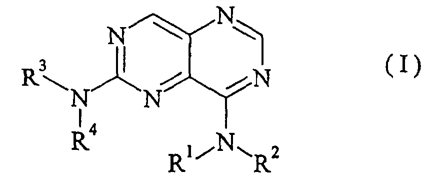 wherein
R¹ denotes a hydrogen atom or a C₁-C₆ alkyl group,
R² denotes an optionally substituted C₆-C₁₀ aryl group,
R³ denotes a hydrogen atom or a C₁-C₆ alkyl group, and
R⁴ denotes a hydrogen atom or an optionally substituted C₁-C₆ alkyl, C₃-C₆ alkenyl, C₃-C₈ cycloalkyl or a 4- to 7-membered, nitrogen-containing heterocyclyl group, or
R³ and R⁴ together with the nitrogen atom linked to them denote an optionally substituted heterocyclyl group,
**characterised in that** the compound of formula II, 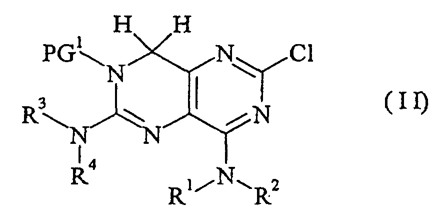 wherein R¹, R², R³, and R⁴ are as herein defined and
PG¹ denotes a suitable protecting group,
(a) after the protecting group has been cleaved,
(b) is treated with a reducing agent, and
(c) is treated with an oxidising agent.

2. Process according to claim 1, **characterised in that** the protecting group is a C₁₋₆ alkylcarbonyl group and it is cleaved by treating with an aqueous base.

3. Process according to claim 1 or 2, **characterised in that** in step (b) the compound obtained after the protecting group has been cleaved is hydrogenated in the presence of a transition metal catalyst or treated with hydrogen iodide, optionally in the presence of phosphorus.

4. Process according to one of claims 1 to 3, **characterised in that** in step (c) the compound obtained in step (b) is treated with a peroxodisulphate or hydrogen peroxide.

5. Process for preparing a compound of formula II according to claim 1, **characterised in that** a compound of formula III, 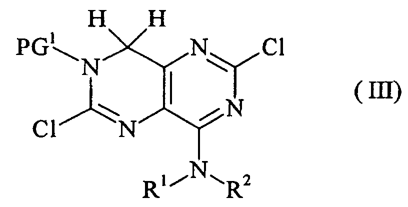 wherein R¹, R² and PG¹ are as herein defined,
is reacted with an amine of formula IV,
R³R⁴NH (IV)
wherein R³ and R⁴ are as herein defined,
optionally in the presence of a base.

6. Process according to claim 5, **characterised in that** the reaction is carried out in the presence of a tertiary amine.

7. Process according to claim 5 or 6, **characterised in that** the reaction is carried out in a temperature range of 0°C to 150°C.

8. Process for preparing a compound of formula III according to claim 5, **characterised in that** a compound of formula V, 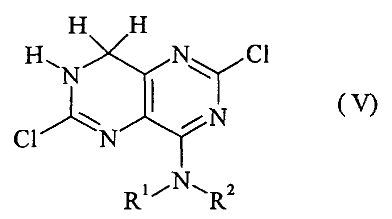 wherein R¹ and R² are as herein defined, is provided with a suitable protecting group (PG¹).

9. Process according to claim 8, **characterised in that** the compound of formula V is reacted with a C₁₋₆ carboxylic acid or a reactive derivative thereof in the presence of a base.

10. Process according to claim 8 or 9, **characterised in that** the compound of formula V is reacted with a C₁₋₆ carboxylic acid chloride in the presence of a tertiary amine.

11. Process for preparing a compound of formula V according to claim 8, **characterised in that** a compound of formula VI, 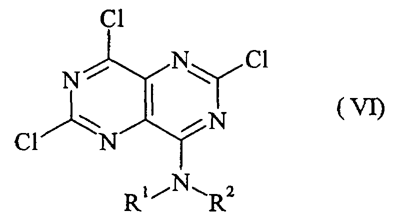 wherein R¹ and R² are as herein defined, is reduced.

12. Process according to claim 11, **characterised in that** the reduction is carried out with a boranate.

13. Process according to claim 12, **characterised in that** the reduction is carried out with sodium boranate in the presence of a base and a polar solvent.

14. Process for preparing a compound of formula VI according to claim 11, **characterised in that** 2,4,6,8-tetrachloropyrimido[5,4,*d*]pyrimidine is reacted with an amine of formula VIII,
R¹R²NH (VIII)
wherein R¹ and R² are as herein defined.

15. 2-Chloro-4,6-diamino-7,8-dihydropyrimido[5,4-*d*]pyrimidine of formula II 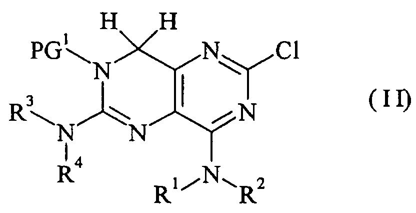 wherein R¹, R², R³, and R⁴ are as herein defined and
PG¹ denotes a C₁₋₆ alkanoyl group.

16. 4-Amino-2,6-dichloro-7,8-dihydropyrimido[5,4-*d*]pyrimidine of formula III 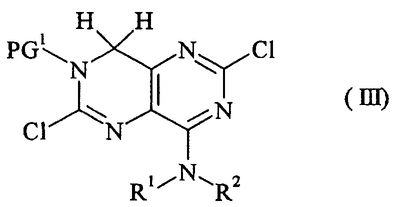 wherein R¹ and R² are as herein defined, and
PG¹ denotes a C₁₋₆ alkanoyl group.

17. 4-Amino-2,6-dichloro-7,8-dihydropyrimido[5,4-*d*]pyrimidine of formula V, 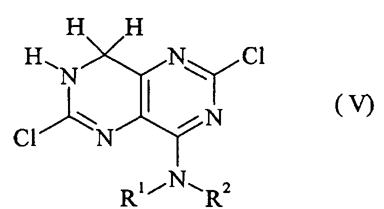 wherein R¹ and R² are as herein defined.

18. 4-Amino-2,6,8-trichloropyrimido[5,4-*d*]pyrimidine of formula VI, 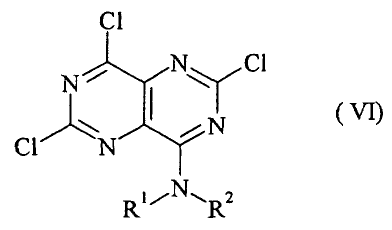 wherein R¹ and R² are as herein defined.

## Revendications

1. Procédé de préparation de 4,6-diaminopyrimido[5,4-*d*]pyrimidines de formule I 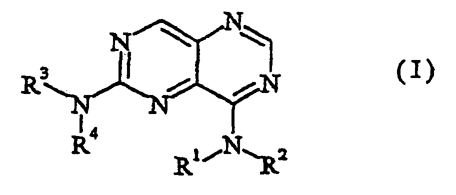 où
R¹ représente un atome d'hydrogène ou un groupement C₁-C₆-alkyle,
R² représente un groupement C₆-C₁₀-aryle éventuellement substitué,
R³ représente un atome d'hydrogène ou un groupement C₁-C₆-alkyle, et
R⁴ représente un atome d'hydrogène ou un groupement C₁-C₆-alkyle, C₃-C₆-alcényle, C₃-C₈-cycloalkyle éventuellement substitué ou un groupement hétérocyclyle azoté à 4 à 7 chaînons, ou
R³ et R⁴ représentent avec l'atome d'azote auquel ils sont liés un groupement hétérocyclyle éventuellement substitué,
**caractérisé en ce que** l'on traite le composé de formule II 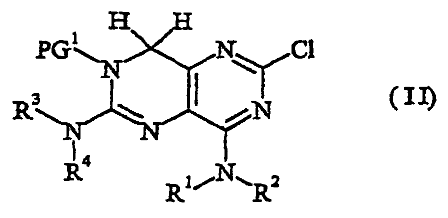 où R¹, R², R³ et R⁴ possèdent la signification indiquée et
PG¹ représente un groupe protecteur approprié,
(a) après clivage du groupe protecteur,
(b) avec un réducteur, et
(c) avec un oxydant.

2. Procédé selon la revendication 1 **caractérisé en ce que** le groupe protecteur est un groupe C₁-C₆-alkylcarbonyle et on le clive par traitement avec une base aqueuse.

3. Procédé selon la revendication 1 ou 2 **caractérisé en ce que**, dans l'étape (b), on hydrogène le composé obtenu après clivage du groupe protecteur en présence d'un catalyseur de type métal de transition ou on le traite avec de l'iodure d'hydrogène éventuellement en présence de phosphore.

4. Procédé selon l'une des revendications 1 à 3 **caractérisé en ce que**, dans l'étape (c), on traite le composé obtenu dans l'étape (b) avec un peroxodisulfate ou le peroxyde d'hydrogène.

5. Procédé de préparation d'un composé de formule II selon la revendication 1 **caractérisé en ce que** l'on fait réagir un composé de formule III 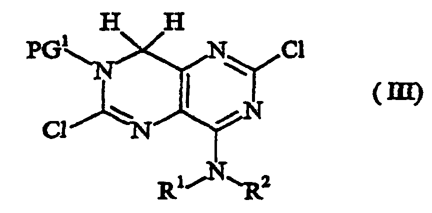 où R¹, R² et PG¹ possèdent les significations indiquées,
avec une amine de formule IV
R³R⁴NH (IV)
où R³ et R⁴ possèdent les significations indiquées,
éventuellement en présence d'une base.

6. Procédé selon la revendication 5 **caractérisé en ce que** l'on conduit la réaction en présence d'une amine tertiaire.

7. Procédé selon la revendication 5 ou 6 **caractérisé en ce que** l'on conduit la conversion dans un domaine de température de 0°C à 150°C.

8. Procédé de préparation d'un composé de formule III selon la revendication 5 **caractérisé en ce que** l'on munit un composé de formule V 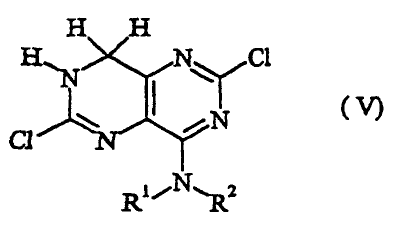 où R¹ et R² présentent la signification indiquée d'un groupe protecteur (PG¹) approprié.

9. Procédé selon la revendication 8 **caractérisé en ce que** l'on fait réagir le composé de formule V avec un acide C₁₋₆-carboxylique ou un dérivé réactif de celui-ci en présence d'une base.

10. Procédé selon la revendication 8 ou 9 **caractérisé en ce que** l'on fait réagir le composé de formule V avec un chlorure d'acide C₁₋₆-carboxylique en présence d'une amine tertiaire.

11. Procédé de préparation d'un composé de formule V selon la revendication 8 **caractérisé en ce que** l'on réduit un composé de formule VI 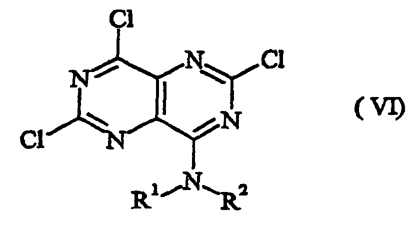 où R¹ et R² présentent la signification indiquée.

12. Procédé selon la revendication 11 **caractérisé en ce que** l'on conduit la réduction avec un boranate.

13. Procédé selon la revendication 12 **caractérisé en ce que** l'on conduit la réduction avec le boranate de sodium en présence d'une base et d'un solvant polaire.

14. Procédé de préparation d'un composé de formule VI selon la revendication 11 **caractérisé en ce que** l'on fait réagir la 2,4,6,8-tétrachloropyrimido[5,4-*d*]pyrimidine avec une amine de formule VIII
R¹R²NH (VIII)
où R¹ et R² présentent la signification indiquée.

15. 2-chloro-4,6-diamino-7,8-dihydropyrimido[5,4-*d*]pyrimidine de formule II 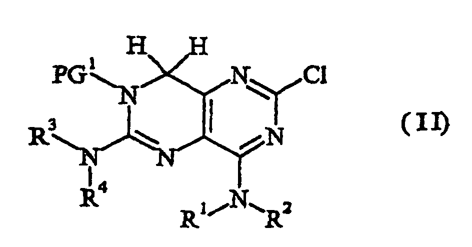 où R¹, R², R³ et R⁴ possèdent la signification indiquée et
PG¹ représente un groupe C₁₋₆-alcanoyle.

16. 4-amino-2,6-dichloro-7,8-dihydropyrimido[5,4-*d*]pyrimidine de formule III 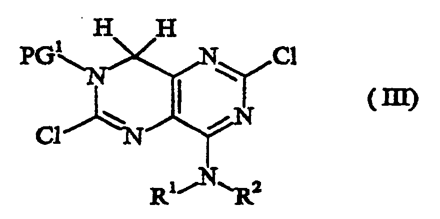 où R¹ et R² possèdent les significations indiquées et
PG¹ représente un groupe C₁₋₆-alcanoyle.

17. 4-amino-2,6-dichloro-7,8-dihydropyrimido[5,4-*d*]pyrimidine de formule V 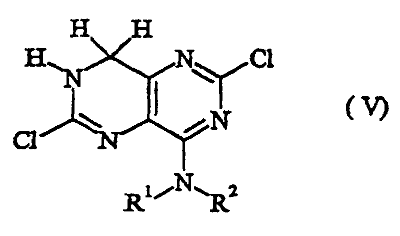 où R¹ et R² possèdent la signification indiquée.

18. 4-amino-2,6,8-trichloropyrimido[5,4-*d*]pyrimidine de formule VI 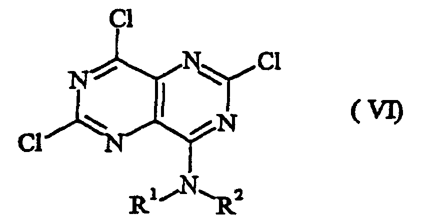 où R¹ et R² présentent la signification indiquée.
